# EUROPEAN PATENT APPLICATION

(11) **EP 4 166 540 A1**
(43) Date of publication of application: **19.04.2023**
(21) Application number: 20940573.7
(22) Date of filing: 15.06.2020
(51) Int. Cl.: C07D 207/27, C07C 237/06

(54) **PREPARATION METHOD FOR BRIVARACETAM**

(71) Applicant: Zhejiang Tianyu Pharmaceutical Co., Ltd, Taizhou, Zhejiang 318020 (CN)
(72) Inventor: WANG, Zhen, Taizhou, Zhejiang 318020 (CN); KE, Chunlong, Taizhou, Zhejiang 318020 (CN); LIU, Junfeng, Taizhou, Zhejiang 318020 (CN); ZHANG, Peng, Taizhou, Zhejiang 318020 (CN); LI, Qichao, Taizhou, Zhejiang 318020 (CN); ZHU, Guorong, Taizhou, Zhejiang 318020 (CN); TU, Yongjun, Taizhou, Zhejiang 318020 (CN)
(74) Representative: Kador & Partner Part mbB
(86) International application number: PCT/CN2020/096111
(87) International publication number: WO 2021/253162

(57) **Abstract**

Provided is a preparation method for brivaracetam. The method has a synthetic route as shown below. The preparation method for the brivaracetam is simple, economical, environmentally friendly and suitable for industrialization:

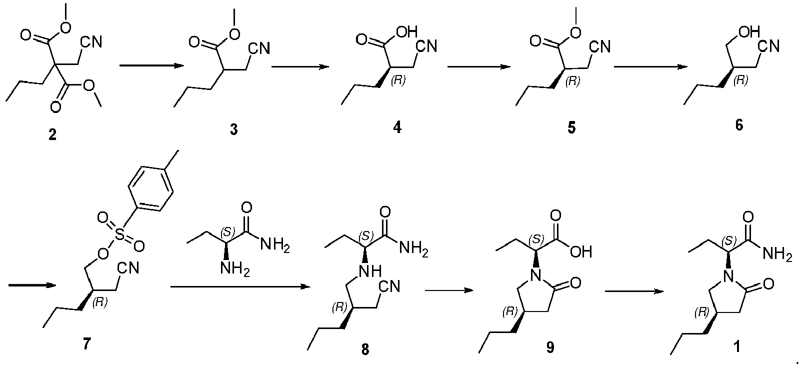

## Description

### TECHNICAL FIELD

The present invention relates to the field of drug synthesis, in particular to a preparation method for brivaracetam.

### BACKGROUNG

Brivaracetam with a chemical name of (S)-2-((R)-2-oxo-4-propylpyrrolidin-1-yl)butanamide has the following chemical structural formula:

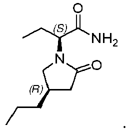

Brivaracetam is a III-generation antiepileptic drug newly developed by drug manufacturer UCB and a novel high-affinity ligand of synaptic vesicle protein 2A (SV2A), and also has a certain inhibitory effect on voltage-dependent sodium channels. In 2016, brivaracetam was approved by FDA for the treatment of epileptic seizures, and the research results show that brivaracetam has a good curative effect on generalized epileptic seizures.

UCB company discloses a preparation method for brivaracetam in literature Org. Process Res. Dev. 2016, 20, 1566-1575. A synthesis route of this method is shown as follows, wherein dimethyl propylmalonate and tert-butyl bromoacetate are condensed and decarboxylated to synthesize tert-butyl β-carbomethoxy hexanoate, the obtained tert-butyl β-carbomethoxy hexanoate is hydrolyzed and resolved under the action of lipase to obtain R-structured tert-butyl β-carboxy hexanoate with a yield of 42%, and then reduced and cyclized to obtain R-structured β-propyl butyrolactone. However, the yield is only 32% when ethyl β-bromomethyl hexanoate is condensed with S-2-amino butanamide in the last step, which causes the whole production cost to be increased.

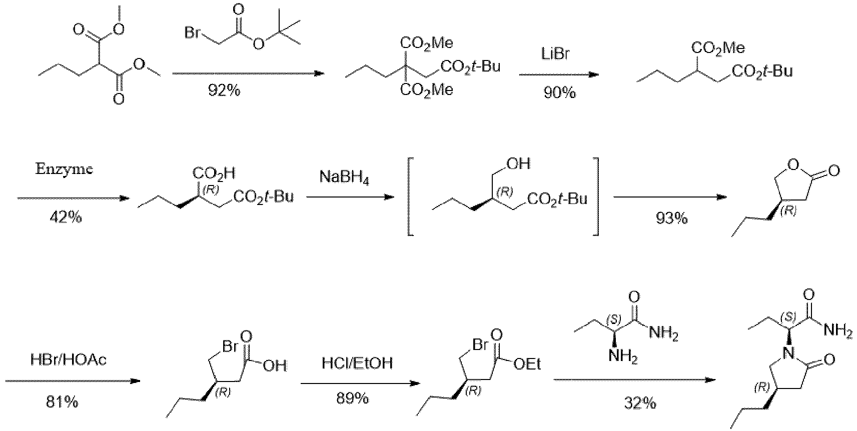

Patent CN108503610 discloses a preparation method for a brivaracetam intermediate. A synthesis route of this method is shown as follows, wherein oxazolinone chiral auxiliary reagent is used to induce ortho-position of valeramide to introduce chiral cyanomethyl, then the corresponding amide is reduced to obtain R-3-hydroxymethylhexanenitril, which is finally acidize and subjected to ring closure to obtain R-structured β-propyl butyrolactone. In this method, R-3-hydroxymethylhexanenitril needs to be obtained by the chiral auxiliary reagent, which needs to be removed after the introduction, so the atomic economy is not high.

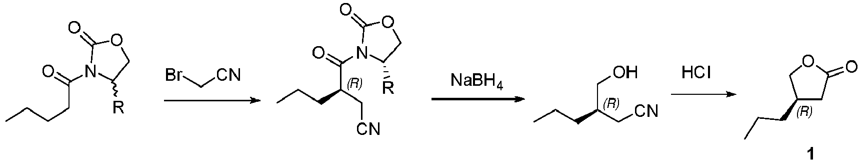

In conclusion, the existing methods for synthesizing brivaracetam through the intermediate R-structured β-propyl butyrolactone mainly have the following shortcomings:
1) The yield of the intermediate R-structured β-propyl butyrolactone obtained by the enzymatic resolution process is very low in the subsequent process of conversion to brivaracetam, resulting in high comprehensive production cost.
2) The synthesis of R-3-hydroxymethylhexanenitrile requires expensive chiral auxiliary reagent oxazolinone, which leads to the increase of the production cost, and the chiral auxiliary reagent needs to be removed in the synthesis process, resulting in low utilization rate of raw materials and low atomic economy.

In order to overcome the shortcomings of the prior art, it is necessary to develop a synthesis method for brivaracetam, which is simpler, more economical, and more environment-friendly and is suitable for industrialization.

### SUMMARY

The object of the present invention is to provide a preparation method for brivaracetam, which is simpler, more economical, and more environment-friendly and is suitable for industrialization to overcome the shortcomings of the prior art, and the method comprises the following steps: 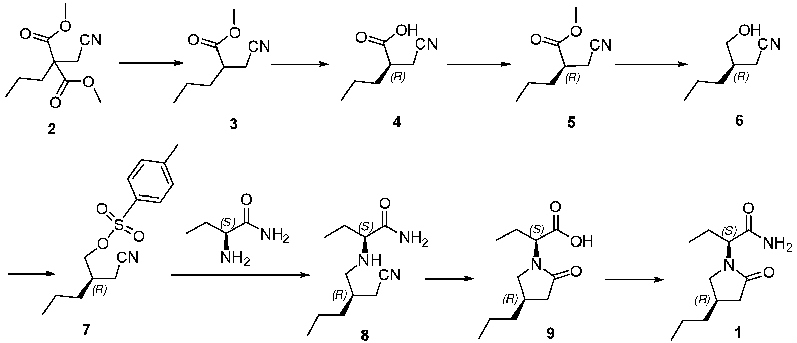
step 1: in an organic solvent, converting compound 2 (dimethyl 2-cyanomethyl-2-propylmalonate) into compound 3 (methyl 2-cyanomethylpentanoate) by removing a methoxycarbonyl group under an action of lithium bromide;
   wherein the organic solvent is selected from DMF and/or DMSO, and preferably DMF;
   a molar ratio of compound 2 to lithium bromide is 1:0.9-1.5, and preferably 1:1.0; and
   a reaction temperature is controlled at 120-150°C, and preferably 130-140°C;
step 2: performing enzymatic hydrolysis on compound 3 under an action of porcine pancreatic lipase to obtain compound 4 (optically pure R-2-cyanomethylpentanoic acid);
   wherein a CAS registration number of the porcine pancreatic lipase is 9001-62-1;
   a mass ratio (g/g) of compound 3 to porcine pancreatic lipase is 1:0.4-1.0, and preferably 1:0.5;
   a reaction pH is controlled at 8.0-8.1; and
   a reaction temperature is controlled at 25-35°C, and preferably 28-33°C;
step 3: subjection compound 4 to a methyl esterification with methanol to obtain compound 5 (methyl R-2-cyanomethylpentanoate) in the presence of acid;
   wherein the acid used is one or more of hydrochloric acid, sulfuric acid and methanesulfonic acid, and preferably 30% aqueous solution of hydrochloric acid;
   a molar ratio of compound 4 to the acid is 1:0.2-0.3, and preferably 1:0.25; and
   a reaction temperature is controlled at 30-50°C, and preferably 35-45°C;
step 4: in an organic solvent, reducing compound 5 into compound 6 (R-2-hydroxymethylhexanenitrile) under an action of sodium borohydride/methanol;
   wherein the organic solvent is selected from tetrahydrofuran, 2-methyltetrahydrofuran and the like, and preferably tetrahydrofuran;
   a molar ratio of compound 6 to sodium borohydride and methanol is 1:1-3:5-9, and preferably 1:2:7;
   wherein a reaction temperature is controlled at 30-60°C, and preferably 40-50°C;
step 5: subjecting compound 6 and para-toluene sulfonyl chloride to sulfonylation to obtain compound 7 in the presence of alkali;
   wherein a molar ratio of compound 6 to para-toluene sulfonyl chloride is 1:1.0-1.5, and preferably 1:1.4;
   the alkali used is selected from one or more of triethylamine, diisopropylethylamine, potassium carbonate and sodium hydroxide, and preferably triethylamine;
   a molar ratio of compound 6 to the alkali is 1:2-4, and preferably 1:3; and
   a reaction temperature is controlled at 15-30°C, and preferably 20-25°C;
step 6: in an organic solvent, condensing compound 7 with S-2-aminobutanaamide to obtain compound 8 in the presence of alkali and tetrabutylammonium iodide;
   wherein the organic solvent is selected from one or more of acetonitrile, dimethylformamide, isopropyl acetate, ethyl acetate and toluene, and preferably acetonitrile;
   the alkali is selected from one or more of sodium carbonate, potassium carbonate, sodium bicarbonate, triethylamine and diisopropylethylamine;
   a molar ratio of compound 7, to the alkali, S-2-aminobutanamide and tetrabutylammonium iodide is 1:2.0-4.0:1.0-2.0:0.2-1.0, and preferably 1:2.3:1.7:0.5; and
   a reaction temperature is controlled at 70-90°C, and preferably 80°C-85°C;
step 7: cyclizing compound 8 in methanol to obtain compound 9 (brivaracetam acid) in the presence of acid;
   wherein the acid used is hydrochloric acid, sulfuric acid, hydrobromic acid, methanesulfonic acid, p-toluenesulfonic acid and the like, and preferably 30% aqueous solution of hydrochloric acid;
   a mass ratio (g/g) of compound 8 to the acid is 1: 10-20, and preferably 1: 15; and
   a reaction temperature is controlled at 40-70°C, and preferably 50-55°C; and
step 8: in an organic solvent, carrying out an amidation reaction between compound 9 and ammonia gas to obtain compound 1 (brivaracetam) in the presence of activator;
   wherein the activator is selected from pivaloyl chloride;
   a molar ratio of compound 9 to the activator is 1: 1.1-1.8, and preferably 1:1.5;
   the organic solvent is selected from dichloromethane, toluene and the like, and preferably dichloromethane; and
   a reaction temperature is controlled at -5°C-15°C, and preferably 0°C-10°C.

The present invention has the beneficial effects that:
(1) According to the present invention, the optically pure R-2-cyanomethylvaleric acid is obtained stereo-specifically through the enzymatic resolution process, with mild reaction conditions and no need for chemical resolution, which is beneficial to the chiral construction of brivaracetam lactam ring.
(2) According to the present invention, the brivaracetam acid can be directly obtained by the acidic cyclization of compound 8, with an outstanding step economy advantage, and high cyclization yield, thus significantly reducing the production cost of raw materials.
(3) The present invention has mild reaction conditions in each step, economical steps and high yield, effectively reduces the cost, and is suitable for industrial production.

### EMBODIMENTS

The present invention is further described hereinafter with reference to the specific embodiments, but the embodiments of the present invention are not limited to these embodiments.

Information of all instruments and reagents of the present invention is as follows:

The solvents and reagents used in the present invention were all purchased from Aladdin Reagent Corporation, and all the reagents were analytically pure; the optical rotation of the present invention was measured on automatic polarimeter MCP5300 of Anton Paar; high-resolution mass spectrometry data were measured by Waters Xevo G2-S QTof high-resolution mass spectrometer; Nuclear magnetic resonance wave spectrum data were measured by Bruker AVANCE III HD 400M; and HPLC purity was measured by Agilent 1260 high performance liquid chromatograph.

### Reference Example: Synthesis of compound 2

Under the protection of nitrogen, dimethyl propyl malonate (300.0 g, 1.72 mol, 1.0 eq), THF (600 mL) and potassium carbonate (276 g, 2 mol, 1.16 eq) were put into a 3L four-necked flask, cooled to -15°C to -5°C, and dropwise added with a solution of bromoacetonitrile (227.3 g, 1.9 mol, 1.1 eq)/THF (300 mL) at -15°C to -5°C for 2-3 hours. After the dropwise adding, the temperature was kept for 1 hour, then heated to 0-10°C, and kept for 1 hour. After the reaction, a saturated NH₄Cl aqueous solution (300 mL) was dropwise added at 0°C-10°C for 0.5 hour to 1 hour. The system was subjected to desolvation under reduced pressure was carried out at 40-45°C to remove THF. After THF was completely removed, CH₂Cl₂ (1.2 L), water (300 mL) and 30 g of activated carbon were added, stirred at 20-25°C for 30 minutes, and then filtered. Organic layers were washed with saturated NH₄Cl aqueous solution twice, with 600 mL of saturated NH₄Cl aqueous solution in each time. Aqueous layers were combined and washed with CH₂Cl₂ twice, with 600 mL of CH₂Cl₂ in each time, and then organic layers were conbined and washed with water twice, with 600 mL of water in each time. The organic layers were subjected to desolvation under reduced pressure to obtain 349.0 g of compound 2 as colorless oil with a yield of 95%. ESI-HRMS (m/z): C₁₀H₁₆NO₄ [M+H⁺], theoretical calculated value: 214.1074, and measured value: 214.1068; ¹HNMR (400MHz, CDCl₃) δ 3.79 (s, 6H), 2.96 (s, 2H), 2.06 (m, 2H), 1.25 (q, *J*=7.2Hz, 2H), 0.98 (t, *J*=7.2Hz, 3H); ¹³CNMR (100Hz, CDCl₃) δ 169.3, 116.3, 11.5, 53.3, 35.0, 21.9, 17.6, 14.0.

### Example 1: Synthesis of compound 3

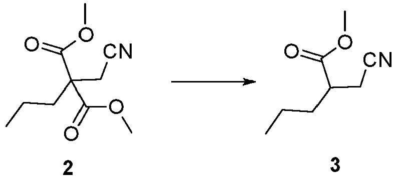

Compound 2 (130 g, 0.61 mol, 1.0 eq), LiBr (53 g, 0.61 mol, 1.0 eq), DMF (650 mL) and water (22.0 g, 1.22 mol, 2.0 eq) were added into a 1L four-necked flask, and heated to 130-140°C. The temperature was kept for 4 hours. After the reaction, the temperature was cooled to 10-20°C. The temperature was controlled at 10-20°C, and saturated NH₄Cl aqueous solution (260 mL) and toluene (780 mL) were added, stirred for 30 minutes, and the system was allowed to settle for layering. Aqueous layers were washed with toluene (400 mL) once, organic layers were combined, washed with water twice, with 390 mL of water in each time, and then the system was allowed to settle for layering. Organic layers were dried with anhydrous sodium sulfate, and then de-solvated under reduced pressure to dryness to obtain 86.7 g of compound 3 as colorless oil with a yield of 92%. ESI-HRMS (m/z): C₈H₁₄NO₂ [M+H⁺], theoretical calculated value: 156.1019, and measured value: 154.1023; ¹HNMR (400MHz, d⁶-acetone) δ 3.70 (s, 3H), 2.82 (m, 1H), 2.73 (m, 2H), 1.70-1.62 (m, 2H), 1.36 (m, 2H), 0.92 (t, *J*=7.2Hz, 3H); ¹³CNMR (100Hz, d⁶-acetone) δ 174.1, 118.9, 52.3, 41.9, 34.2, 20.4, 14.1.

### Example 2: Synthesis of compound 4

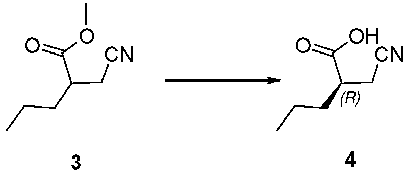

Tris(hydroxymethyl)aminomethane (2.7 g) and water (720 mL) were added into a 2L four-necked flask, stirred and the pH of the thus obtained system was adjusted to about 8.1 with 1.0M HCl, added with porcine pancreatic lipase (45.0 g), heated to 28-33°C, and then added with compound 3 (90.0 g, 0.58 mol, 1.0 eq) prepared according to the method of Example 1, and THF (90mL). The temperature was controlled at 28-33°C, the pH was adjusted to 8.0-8.1 with 1.0M NaOH solution, and the temperature was kept for 14-18 hours. After the reaction, diatomite (45.0 g) was added, stirred for 30 minutes, and then filtered. Filter residues were pulped and filtered with 450 mL of ethyl acetate. Filtrates were combined and layered. Aqueous layers were washed with ethyl acetate thrice, with 450 mL of ethyl acetate in each time, aqueous layers were cooled to 0-5°C. The pH was adjusted to 1.9-2.1 by 1.0M H₂SO₄ (248 g). After the pH was adjusted, the temperature was increased to 20-25°C, and 45 g of diatomite and 900 mL of ethyl acetate were added, stirred for 30 minutes, and filtered. Filtrates were layered, aqueous layers were washed with ethyl acetate twice, with 540 mL of ethyl acetate in each time, and ethyl acetate layers were combined and washed with water thrice, with 900 mL of water in each time. Organic layers were dried with anhydrous sodium sulfate and were subjected to desolvation under reduced pressure. After the desolvation was completed, 32.0 g of compound 4 as colorless oil was obtained, with a yield of 79%. [α]^{D}₂₅: +21.6°(c=1.0g/100mL, chloroform); ESI-HRMS (m/z): C₇H₁₀NO₂ [M-H⁺], theoretical calculated value: 140.0717, and measured value: 140.0722; ¹HNMR (400MHz, CDCl₃) δ 2.86-2.79 (m, 1H), 2.74-2.67 (m, 1H), 2.63-2.54 (m, 1H), 1.85-1.78 (m, 1H), 1.72-1.65 (m, 1H), 1.49-1.38 (m, 2H), 0.95 (t, J=7.2Hz, 3H); ¹³CNMR (100Hz, CDCl₃) δ 117.6, 70.5, 41.1, 33.1, 19.7, 18.9, 13.7.

### Example 3: Synthesis of compound 5

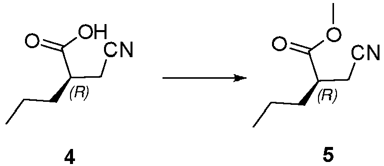

Compound 4 (32.0 g, 0.23 mol, 1.0 eq), methanol (128 mL), refined 30% aqueous solution of hydrochloric acid (5.8 g, 0.058 mol, 0.25 eq) were added into a 250ml four-necked flask, heated to 35-45°C and kept for 20 hours. After the reaction, the methanol was removed under reduced pressure at 40-45°C. After the methanol was removed completely, 320 mL of dichloromethane and 320 ml of water were added, stirred for 10 minutes, and the system was allowed to settle for layering. Aqueous layers were washed once with 1,600 mL of dichloromethane, and the system was allowed to settle for layering. Organic layers were combined, and the organic layers were washed twice with saturated NaCl aqueous solution, then the organic layers were washed once with 320 ml of saturated NaHCO₃ aqueous solution, and the system was allowed to settle for layering. Organic layers were dried with anhydrous sodium sulfate and were subjected to desolvation under reduced pressure to obtain 32.6 g of compound 5 as colorless oil with a yield of 93%. ESI-HRMS (m/z): C₈H₁₄NO₂ [M+H⁺], theoretical calculated value: 156.1019, and measured value: 154.1016; ¹HNMR (400MHz, d⁶-acetone) δ 3.72 (s, 3H), 2.84 (m, 1H), 2.75 (m, 2H), 1.73-1.62 (m, 2H), 1.38 (m, 2H), 0.92 (t, *J*=7.2Hz, 3H); ¹³CNMR (100Hz, d⁶-acetone) δ 174.2, 119.0, 52.4, 42.0, 34.3, 20.5, 14.2.

### Example 4: Synthesis of compound 6

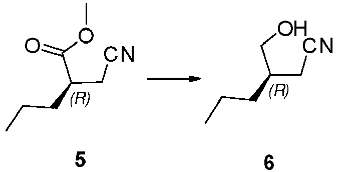

Under the protection of nitrogen, compound 5 (32.5 g, 0.21 mol, 1.0 eq) and THF (325 mL) were added into a four-necked flask. The temperature was controlled at 10-20°C, and then sodium borohydride (15.9 g, 0.42 mol, 2.0 eq) was added in batches. After addition, the temperature was kept for 30 minutes, and then heated to 30-40°C, and methanol (46.9 g, 1.46 mol, 7 eq) was dropwise added. After the dropwise adding, the temperature was heated to 40-50°C and kept for 4-5 hours. After the reaction, the temperature was reduced to 0-10°C, and 1.0M HCl solution was dropwise added to adjust the pH of the thus obtained system to 7.3-7.9, which was unchanged after repetition measurement. The material liquid was subjected to desolvation under reduced pressure at 40-50°C. After the desolvation was completed, 320 mL of water was added, stirred with 320 mL of ethyl acetate for 20 minutes, and then filtered. Filtrates were allowed to settle for layering, aqueous layers were washed with 320 mL of ethyl acetate once, ethyl acetate layers were combined and washed with water for thrice, with 320 mL of water in each time, then the system was allowed to settle for layering. Ethyl acetate layers were dried with anhydrous sodium sulfate and were subjected to desolvation under reduced pressure. After the desolvation was completed, 20.7 g of compound 6 as colorless oil was obtained, with a yield of 78%. [α]^{D}₂₅: +13.6°(c=1.0g/100mL, methanol); ESI-HRMS (m/z): C₇H₁₄NO [M+H⁺], theoretical calculated value: 128.1070, and measured value: 128.1075; ¹HNMR (400MHz, CDCl₃) δ: 3.72 (dd, J=10.8, 4.4Hz, 1H), 3.55 (dd, J=10.8, 7.5Hz, 1H), 2.49 (d, J=5.8Hz, 2H), 2.01 (s, 1H), 1.97-1.85 (m, 1H), 1.51-1.30 (m, 4H), 0.94 (dd, J=9.5, 4.0Hz, 3H).

### Example 5: Synthesis of compound 7

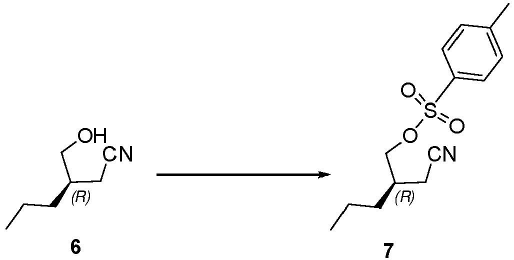

Under the protection of nitrogen, compound 6 (16.0 g, 0.12 mol, 1.0 eq) and dichloromethane (160 mL) were added into a four-necked flask. The temperature was controlled at 0-5°C, and then triethylamine (38.2 g, 0.38 mol, 3.0 eq) was dropwise added for 0.5-1 hour. After the dropwise adding, the temperature was kept for 30 minutes, and a dichloromethane (160 mL) solution containing para-toluene sulfonyl chloride (33.6 g, 0.18 mol, 1.4 eq) was dropwise added for 1-2 hours. After the dropwise adding, the temperature was heated to 20-25°C and kept for 12-16 hours. After the reaction, the temperature was cooled to 0-10°C, an aqueous solution of sodium bicarbonate (15.0 g of sodium bicarbonate and 200 mL of water) was added, heated to 20-25°C and kept for 30 minutes, and then the system was allowed to settle for layering. Aqueous layers were washed once with 160 mL of dichloromethane, organic layers were combined and washed with with aqueous solution of sodium bicarbonate thrice, with 15.0 g of sodium bicarbonate and 200 mL of water in each time. Organic layers were washed with water twice, with 160 mL of water in each time. After the washing was completed, the organic layers were dried with anhydrous sodium sulfate and were subjected to desolvation under reduced pressure. After the desolvation was completed, 33.6 g of compound 7 as colorless oil was obtained, with a yield of 95%. [α]^{D}₂₅: +17.3° (c=1.0g/100mL, chloroform); ESI-HRMS (m/z): C₁₄H₂₀NO₃S [M+H⁺], theoretical calculated value: 282.1158, and measured value: 282.1165; ¹HNMR (400MHz, CDCl₃) δ: 7.79 (m, 2H), 7.36 (m, 2H), 4.07 (m, 1H), 3.92 (m, 1H), 2.43 (s, 3H), 2.39 (m, 2H), 2.12 (m, 1H), 1.38 (m, 2H), 1.29 (m, 2H), 0.88 (m, 3H); ¹³C NMR (CDCl₃, 100Hz) δ: 145.2, 132.4, 130. 0, 127.9, 117.4, 70.5, 35.0, 31.8, 21.6, 19.6, 19.2, 13.8.

### Example 6: Synthesis of compound 8

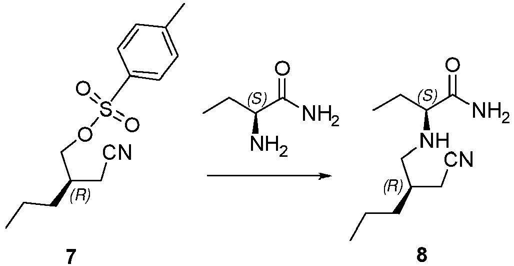

Under the protection of nitrogen, compound 7 (10.4 g, 37 mmol, 1.0 eq), tetrabutylammonium iodide (6.8 g, 18.5 mmol), sodium carbonate (8.7 g, 82.1 mmol, 2.3 eq), S-2-aminobutanamide (6.6 g, 64.6 mmol, 1.7 eq) and acetonitrile (85 mL) were added into a four-necked flask, heated to 80-85°C, and refluxed. Then, the temperature was kept for 4-5 hours. After the reaction was completed, the temperature was reduced to 10-20°C, and then the mixture was filtered. Residues were rinsed with isopropyl acetate (85 mL), filtrates were washed with water thrice, with 85 mL of water in each time, and organic layers were dried with anhydrous sodium sulfate and were subjected to desolvation under reduced pressure. After the desolvation was completed, 6.9 g of compound 8 as colorless oil was obtained, with a yield of 88%. [α]^{D}₂₅: -16.7° (c=1.0g/100mL, chloroform); ESI-HRMS (m/z): C₁₁H₂₂N₃O [M+H⁺], theoretical calculated value: 212.1757, and measured value: 212.1752; ¹HNMR (400MHz, CDCl₃) δ: 6.73 (s, 1H), 6.42 (s, 1H), 2.88 (m, 1H), 2.62 (m, 1H), 2.51-2.32 (m, 3H), 1.75 (m, 1H), 1.62-1.51 (m, 2H), 1.34-1.21 (m, 5H), 0.89-0.82 (m, 6H). ¹³C NMR (CDCl₃, 100Hz) δ: 177.5, 118.7, 64.1, 51.1, 35.5, 33.5, 26.2, 19.9, 19.6, 9.9, 7.5.

### Example 7: Synthesis of compound 8

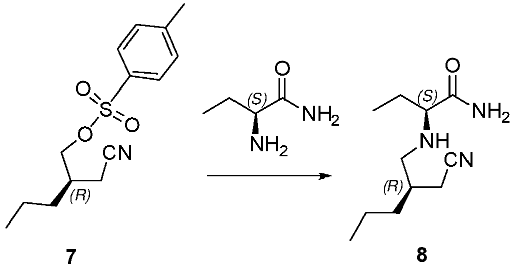

Under the protection of nitrogen, compound 7 (10.4 g, 37 mmol, 1.0 eq), tetrabutylammonium iodide (6.8 g, 18.5 mmol), diisopropylethylamine (10.6 g, 82.1 mmol, 2.3 eq), (S)-2-aminobutanamide (6.6 g, 64.6 mmol, 1.7 eq) and acetonitrile (85 mL) were added into a four-necked flask, heated to 80-85°C, and refluxed. Then, the temperature was kept for 4-5 hours. After the reaction, the temperature was reduced to 10-20°C, and 100 mL of water and 100 mL of ethyl acetate were added. After layering, organic layers were dried with anhydrous sodium sulfate and were subjected to desolvation under reduced pressure. After the desolvation was completed, 6.3 g of compound 8 as colorless oil was obtained, with a yield of 80%. Characterization data of the obtained oil were consistent with that of Example 6.

### Example 8: Synthesis of compound 9

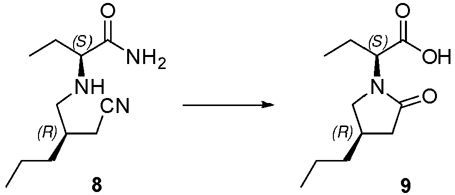

Under the protection of nitrogen, compound 8 (11.6 g, 55 mmol, 1.0 eq), refined 30% aqueous solution of hydrochloric acid (87 mL) and methanol (116 mL) were added into a four-necked flask, heated to 50-55°C, and kept for 16 hours. After the reaction, the temperature was reduced to 0-10°C, and 20% sodium hydroxide solution was dropwise added until the pH of the thus obtained system reached 12-14. Then, the temperature was heated to 20-25°C, and kept for 4 hours. After the mixture was concentrated under reduced pressure, 300 mL of water were added in residues, and extracted with dichloromethane thrice, with 200 mL of dichloromethane in each time. Aqueous layers were adjusted with 1M hydrochloric acid till the pH reached 1-2, and extracted with dichloromethane thrice, with 300 mL of dichloromethane in each time. The combined dichloromethane layers were dried with anhydrous sodium sulfate and dichloromethane was removed under reduced pressure. After drying in vacuum, 9.3 g of compound 9 as white solid was obtained, with a yield of 80%. [α]^{D}₂₅: -26.1° (c=1.0g/100mL, chloroform); ESI-HRMS (m/z): C₁₁H₂₀NO₃ [M+H⁺], theoretical calculated value: 214.1438, and measured value: 214.1445; ¹H NMR(CDCl₃, 400MHz) δ: 11.22 (br, 1H), 4.59 (m, 1H), 3.36 (m, 1H), 3.14 (t, J=8.3Hz, 1H), 2.52 (m, 1H), 2.28 (m, 1H), 2.12 (m, 1H), 2.03 (m, 1H), 1.64 (m, 1H), 1.39 (m, 2H), 1.28 (m, 2H), 0.87 (t, 3H), 0.85 (t, 3H); ¹³C NMR (CDCl₃, 100Hz) δ: 175.9, 173.0, 54.3, 48.4, 36.6, 35.4, 31.1, 20.9, 19.6, 13.0, 9.8.

### Examplet 9: Synthesis of brivaracetam

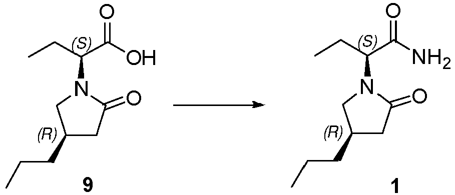

Under the protection of nitrogen, compound 9 (10 g, 47 mmol, 1.0 eq) prepared according to the method of Example 8, dichloromethane (100 mL) and triethylamine (11.9 g, 0.0118 mol, 2.5 eq) were added into a four-necked flask, cooled to 0-5°C, and then pivaloyl chloride (8.5 g, 71 mmol, 1.5 eq) was dropwise added. After the reaction, ammonia gas was introduced to saturation. The temperature was kept at 0-10°C for 6 hours. After the reaction, the mixture was filtered, filtrates were washed with water thrice, with 300 mL of water in each time, and dichloromethane layers were dried with anhydrous sodium sulfate and were subjected to desolvation under reduced pressure. The obtained residues were recrystallized with isopropyl acetate and cyclohexane (1:3) to obtain 8.8 g of compound 1, i.e., brivaracetam, with a yield of 88% and a purity of 99.9 % (HPLC chromatographic conditions: chromatographic column: Inertsil ODS 3V (manufacturer: GL-Science), (250 x 4.6) mm, 5µ. Mobile phase A: buffer: 0.1% perchloric acid aqueous solution; Mobile phase B: acetonitrile: buffer (0.1% perchloric acid aqueous solution) (90:10, V/V); diluent: water: acetonitrile (60:40, V/V); flow rate: 1.0 mL/min; detection wavelength: 215 nm), chiral purity de%: 99.2% (chiral HPLC chromatographic conditions: chromatographic column: Chiralpak AD-H (Make: Daicel), (250 x 4.6) mm, 5 µ. Mobile phase: n-hexane: isopropanol: diethylamine (850:150:0.2, V/V/V). Diluent: n-hexane: isopropanol (80:20, V/V); flow rate: 1.0 mL/min. Detection wavelength: 215 nm.). mp 74~76°C; [α]^{D}₂₅: -60.2° (c=1.0g/100mL, methanol); ESI-HRMS (m/z): C₁₁H₂₁N₂O₂ [M+H⁺], theoretical calculated value: 213.1598, and measured value: 213.1594; ¹H NMR (CDCl₃, 400MHz) δ: 6.71 (s, 1H), 6.14 (s, 1H), 4.51 (dd, J=9.0, 6.4Hz, 1H), 3.61 (dd, J=9.6, 8.0Hz, 1H), 3.00 (dd, J=9.7, 6.7Hz, 1H), 2.53 (dd, J=14.8, 8.5Hz, 1H), 2.39 (m, 1H), 2.12 (dd, J=16.5, 7.8Hz, 1H), 2.03 (m, 1H), 1.70 (m, 1H), 1.44-1.24 (m, 4H), 0.94 (t, 3H), 0.90 (t, 3H); ¹³CNMR (CDCl₃, 100Hz) δ: 175.3, 172.7, 55.7, 49.4, 37.7, 37.0, 31.6, 21.1, 20.4, 13.7, 10.2.

Various embodiments of the present invention have been described above, and the above description is exemplary, not exhaustive, and is not limited to the disclosed embodiments. Many modifications and variations will be apparent to those of ordinary skilled in the art without departing from the scope and spirit of the illustrated embodiments.

## Claims

1. A preparation method for brivaracetam, comprising the following steps: 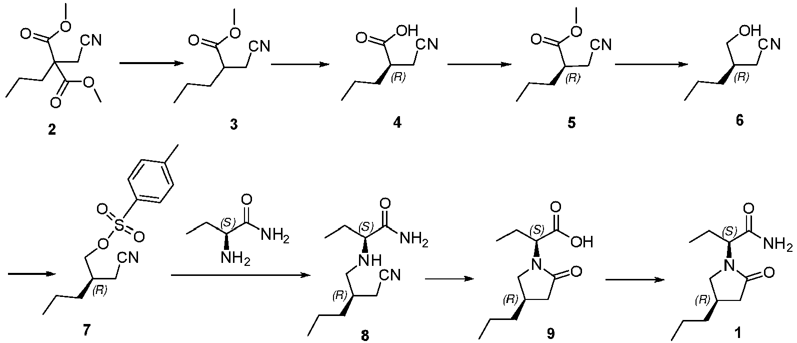
step 1: in an organic solvent, converting compound 2 into compound 3 by removing a methoxycarbonyl group under an action of lithium bromide;
step 2: performing enzymatic hydrolysis on compound 3 under an action of porcine pancreatic lipase to obtain compound 4;
step 3: subjecting compound 4 to a methyl esterification with methanol to obtain compound 5 in the presence of acid;
step 4: reducing compound 5 into compound 6 under an action of sodium borohydride/methanol;
step 5: subjecting compound 6 to sulfonylation with para-toluene sulfonyl chloride to obtain compound 7 in the presence of alkali;
step 6: condensing compound 7 with S-2-aminobutanamide to obtain compound 8 in the presence of alkali and tetrabutylammonium iodide;
step 7: cyclizing compound 8 in methanol to obtain compound 9 in the presence of acid; and
step 8: carrying out an amidation reaction between compound 9 and ammonia gas to obtain compound 1 in the presence of activator.

2. The method according to claim 1, wherein in step 1, the organic solvent is selected from DMF and/or DMSO, and preferably DMF; and a reaction temperature is controlled at 120°C-150°C, and preferably 130°C-140°C.

3. The method according to claim 1, wherein in step 2, a CAS registration number of the porcine pancreatic lipase is 9001-62-1; and a reaction pH is controlled at 8.0-8.1.

4. The method according to claim 1, wherein in step 3, the acid used is one or more of hydrochloric acid, sulfuric acid and methanesulfonic acid, and preferably 30% aqueous solution of hydrochloric acid.

5. The method according to claim 1, wherein in step 4, a molar ratio of compound 6 to sodium borohydride and methanol is 1:1-3:5-9, and preferably 1:2:7.

6. The method according to claim 1, wherein in step 5, the alkali used is selected from one or more of triethylamine, diisopropylethylamine, potassium carbonate and sodium hydroxide, and preferably triethylamine.

7. The method according to claim 1, wherein in step 6, the alkali is selected from one or more of sodium carbonate, potassium carbonate, sodium bicarbonate, triethylamine and diisopropylethylamine; a molar ratio of compound 7, to the alkali, S-2-aminobutanamide and tetrabutylammonium iodide is 1:2.0-4.0:1.0-2.0:0.2-1.0, and preferably 1:2.3: 1.7:0.5; and a reaction temperature is controlled at 70°C-90°C, and preferably 80°C-85°C.

8. The method according to claim 1, wherein in step 7, a reaction temperature is controlled at 40°C-70°C, and preferably 50°C-55°C.

9. The method according to claim 1, wherein in step 8, the activator is selected from pivaloyl chloride.

10. A brivaracetam intermediate 8 represented by the following formula:
